# EUROPEAN PATENT APPLICATION

(11) **EP 1 961 765 A1**
(43) Date of publication of application: **27.08.2008**
(21) Application number: 06025423.2
(22) Date of filing: 08.12.2006
(51) Int. Cl.: C07K 14/635, A61K 38/29

(54) **Truncated PTH peptides with a cyclic conformation**

(71) Applicant: Zealand Pharma A/S, 2600 Glostrup (DK)
(72) Inventor: The designation of the inventor has not yet been filed
(74) Representative: Kiddle, Simon John

(57) **Abstract**

The present invention provides PTH peptides which are cyclized substitution analogues of the truncated PTH fragment PTH(1-17) and which preferably retain the desired or similar biological activity of human PTH (1-34).

## Description

### FIELD OF THE INVENTION

The present invention relates to the substitution analogues of peptide PTH(1-17) with a cyclic structural feature. These polypeptide analogs are useful in the treatment of patients with bone loss. Bone loss may result from conditions such as osteoporosis, glucocorticoid-induced bone loss, hypercortisolism (both subclinical and clinical), cancer, hypercalcemia, renal failure or other kidney disorders, renal transplant and accompanying pharmacological treatments, cholestatic liver diseases, viral hepatitis, bone loss caused by liver transplant,hyperparathyroid disease, bronchial asthma (including hormone-dependent), disorders due to haemodialysis, and osteomalacia. Often bone loss is attributed to idiopathic osteoporosis.

### BACKGROUND OF THE INVENTION

### Parathyroid hormone:

Parathyroid hormone (PTH), an 84 amino acid peptide, is the principal regulator of ionized blood calcium in the human body (Kronenberg, H. M., et al., In Handbook of Experimental Pharmacology, Mundy, G. R., and Martin, T. J., (eds), pp. 185-201, Springer-Verlag, Heidelberg (1993)). PTH is also anabolic to bone when administered intermittently (Rosen, Trends Endocrinol. Metab 2004, 15: 229-33.; Martin et. al., Ann. N. Y. Acad. Sci. 2006, 1068: 458-70.). The osteoblast PTH receptor (PTH receptor 1) can transduce signals through Gs and Gq-proteins to adenylate cyclase and phospholipase C, respectively. Activation of adenylate cyclase is a prerequisite for PTH and its fragments' anabolic action on bone. Whether phospholipase C or other signaling pathways need to be activated, is still unresolved (Murrills et. al., Bone 2004, 35: 1263-72.). Regulation of calcium concentration is necessary for the normal function of the gastrointestinal, skeletal, neurologic, neuromuscular, and cardiovascular systems. PTH synthesis and release are controlled principally by the serum calcium level; a low level stimulates and a high level suppresses both hormone synthesis and release. PTH, in turn, maintains the serum calcium level by directly or indirectly promoting calcium entry into the blood at three sites of calcium exchange: gut, bone, and kidney. PTH contributes to net gastrointestinal absorption of calcium by favouring the renal synthesis of the active form of vitamin D. PTH promotes calcium resorption from bone indirectly by stimulating differentiation of the bone-resorbing cells, osteoclasts. It also mediates at least three major effects in the kidney: stimulation of tubular calcium reabsorption, enhancement of phosphate clearance, and promotion of an increase in the enzyme that completes synthesis of the active form of vitamin D. PTH is thought to exert these effects primarily through receptor-mediated activation of adenylate cyclase and/or phospholipase C.

### Osteoporosis:

Disruption of calcium homeostasis may produce many clinical disorders (e.g., severe bone disease, anemia, renal impairment, ulcers, myopathy and neuropathy) and usually results from conditions that produce an alteration in the level of parathyroid hormone. Hypercalcemia is a condition that is characterized by an elevation in the serum calcium level. It is often associated with primary hyperparathyroidism in which an excess of PTH production occurs as a result of a parathyroid gland lesion (e.g., adenoma, hyperplasia, or carcinoma). Another type of hypercalcemia, humoral hypercalcemia of malignancy (HHM) is the most common paraneoplastic syndrome. It appears to result in most instances from the production by tumours (e.g., squamous, renal, ovarian, or bladder carcinomas) of a class of protein hormone which shares amino acid homology with PTH. These PTH-related proteins (PTHrP) appear to mimic certain of the renal and skeletal actions of PTH and are believed to interact with the PTH receptor in these tissues.

Postmenopausal osteoporosis is a skeletal disorder characterized by a reduction in bone density and strength, associated with an increased risk of fracture (Lane et. al., Clin. Orthop. Relat Res. 2000, 139-50.; Christiansen, Bone 1995, 17: 5135-6S.). Osteoporotic fractures most often occur in the vertebrae, the hips or the femoral neck. These fractures severely impair the patients' quality of life because of pain, long-lasting immobility, and poor recovery.

Bone is a highly active tissue in the human body. Bone is continuously remodelled by two types of cells: bone resorbing osteoclasts, and bone forming osteoblasts. When bone resorption exceeds bone formation, bone loss occurs that may develop into osteoporosis (Seeman and Delmas, N. Engl. J. Med. 2006, 354: 2250-61.).

Osteoporosis is frequently first diagnosed when a fracture has occurred.

Postmenopausal estrogen deficiency is the most common cause of the disease, as estrogen puts a break on osteoclast lifespan. Other major risk factors in the development of osteoporosis include: low calcium intake, vitamin D deficiency, type 1 diabetes, rheumatoid arthritis, long-term use of medication such as anticonvulsants and corticosteroids, and low levels of testosterone in men.

### PTH analogues:

Currently, PTH(1-34), and more recently PTH(1-84), have been brought to the market as bone anabolic drugs used for the treatment of severe osteoporosis.

Although treatment with PTH(1-84) and PTH(1-34) stimulates bone strength and prevents fractures, tolerability is hampered by transient mobilization of calcium and hypercalcemia following each dosing which is commonly associated with nausea. Furthermore, these peptides are not orally or transmucally available, but have to be injected daily.

Synthetic PTH(1-34) exhibits full bioactivity in most cell-based assay systems, has potent anabolic effects on bone mass in animals and has recently been shown to reduce the risk of bone fracture in postmenopausal osteoporotic women (Neer, R. M., et al., N.E.J.M. 344:1434-1441 (2001); Dempster, D. W., et al., Endocr Rev 14:690-709 (1993)). PTH acts on the PTH/PTHrP receptor (P1R), a class II G protein-coupled heptahelical receptor that couples to the adenylyl cyclase/CAMP and phospolipase C/inositol phosphate (IP) signaling pathway (Rippner, H., et al., Science 254:1024-1026 (1991)). Deletion analysis studies have shown that the amino-terminal residues of PTH play a crucial role in stimulating the P1 R to activate the cAMP and IP signaling pathways (Tregear, G. W., et al., Endocrinology 93:1349-1353 (1973); Takasu, H., et al., Biochemistry 38:13453-13460(1999)). Crosslinking and receptor mutagenesis studies have indicated that residues in the amino-terminal portion of PTH interact with the extracellular loops and extracellular ends of the seven transmembrane helices, which reside within the juxtamembrane region of the receptor (Bergwitz, C., et al., J. Biol. Chem. 271:26469-26472 (1996); Hoare, S. R. J., et al., J. Biol. Chem 276:7741-7753 (2001); Behar, V., et al., J. Biol. Chem. 275:9-17 (1999); Shimizu, M., et al., J. Biol. Chem. 275:19456-19460(2000); Luck, M. D., et al., Molecular Endocrinology 13:670-680 (1999)).

PTH(1-31) and its cyclic analogue Ostabolin C have been shown to possess adenylate cyclase-stimulating activity and to retain anabolic actions on bone without affecting blood calcium levels (Whitfield et. al., Calcif. Tissue Int. 1997, 61: 322-6.; Fraher et. al., J. Clin. Endocrinol. Metab 1999, 84: 2739-43.). In WO 04/067021 a lactam bridge between residue 6 and residue 10 of a series of C-terminally truncated PTH(1-34 to 1-10) analogues is proposed to improve the biological activity of the truncated peptide. Further, in DE 19508672 a PTH(1-34) fragment cyclized between position 13 and 17 is proposed but no improves effect is shown.

In WO 03/009804 and WO 04/093902, it is proposed that introduction of α-helix stabilizing amino acids in position 1 and 3 of a PTH(1-14) analogue improves the ability of the compounds to stimulate cAMP accumulation. The most potent compound identified was [Ac₅c¹, Aib³, Gln¹⁰, Har¹¹, Ala¹², Trp¹⁴]PTH(1-14) ([Ac₅c¹, Aib³]MPTH(1-14)). The anabolic action of these compounds is, however, not shown.

Thus there still exists an unmet need for a stable and potent PTH analogue with reduced side effects, e.g. analogues retaining anabolic action without affecting the blood calcium level.

### SUMMARY OF THE INVENTION

Broadly, the present invention provides PTH peptides which are cyclized substitution analogues of a truncated PTH fragment, for example PTH(1-17), and which preferably retain the desired or similar biological activity of human PTH (1-34). Further, the invention provides PTH peptides with improved stability and reduced side effects compared to PTH(1-34) or PTH(1-84). The relatively small size of the compound of the present invention as compared with PTH (1-34) may be utilized in formulations for oral, nasal or pulmonal administration.

Throughout this specification, residue positions are numbered relative to the full length wild-type PTH(1-17). Thus, for example, a reference to position 11 should be construed as a reference to the 11th residue from the N-terminus of PTH(1-17).

Broadly, the present application relates to PTH(1-17) peptides which have one or more substitutions relative to wild-type PTH(1-17), and which may have improved properties as compared to wild type PTH(1-17), [Ac₅c¹, Aib³]MPTH(1-14)) or wild type PTH(1-34). e.g. activity towards a particular receptor, selectivity for one receptor over another, chemical/pharmacological stability, etc. These substitutions may comprise a conservative substitution at any amino acid position optionally in combination with at least one non-conservative amino acid substitution. In particular, the present invention relates to a cyclic link between residue A13 and residue A17, e.g. a cyclic link formed between the side chains of the amino acid residues at these positions.

Accordingly, in one aspect, the present invention relates to a substituted PTH(1-17) peptide having the Formula I:
R1-Z1-A1-A2-A3-A4-A5-A6-A7-A8-A9-A10-A11-A12-A13-A14-Leu-A16-A17-Z2-R2
   wherein
   R1 is NH₂, RHN, RR3N, wherein each of R and R3 independently represent C1-4 alkyl (e.g. methyl), acetyl, formyl, benzoyl or trifluoroacetyl;
   A1 is Ac5c, Gly, Ser, Ala or any alpha-helix stabilizing residue;
   A2 is Val or a conservative substitution;
   A3 is Aib, Ala, Ser or any alpha-helix stabilizing residue;
   A4 is Glu or a conservative substitution;
   A5 is Ile or a conservative substitution;
   A6 is Gln, Glu or a conservative substitution;
   A7 is Leu or Phe or a conservative substitution;
   A8 is Met, Leu, Nle, Val or a conservative substitution;
   A9 is His or a conservative substitution;
   A10 is Gln, Glu, Asp, Ala, Val or a conservative substitution;
   A11 is Har, Arg, Ala, Ile, Lys or a conservative substitution;
   A12 is Ala, Arg, His or a conservative substitution;
   A13 is Lys, Orn, Asp, Glu, Cys, Dab or Dpr;
   A14 is Trp, Phe, Leu, Arg, His or a conservative substitution;
   A16 is Asn, Asp, a conservative substitution or absent;
   A17 is, Lys, Orn, Glu, Cys, Asp, Dab or Dpr; and
R2 is OH, OR, NRH, NRR3 or NH₂, wherein each of R and R3 independently represents C1-4 alkyl (e.g. methyl); and
A13 and A17 are linked by one or more covalent bonds; and
Z1 and Z2 are independently absent or a peptide sequence of 1-10 amino acid units selected from the group consisting of Ala, Leu, Met, Glu, Gln, Lys, Dab, Dpr and Orn;
or a dimer, heterodimer or pharmaceutically acceptable salt or derivative thereof.

In a further aspect, the present invention provides a substituted PTH(1-17) analogue peptide having the Formula II:
R1-Z1-A1-Val-A3-Glu-Ile-A6-A7-A8-His-A10-A11-A12-A13-A14-Leu-A16-A17-Z2-R2
   wherein
   R1 is NH₂, RHN, RR3N, wherein each of R and R3 independently represent C1-4 alkyl (e.g. methyl), acetyl, formyl, benzoyl or trifluoroacetyl;
   A1 is Ac5c, Gly, Ser, Ala or any alpha-helix stabilizing residue;
   A3 is Aib, Ala, Ser or any alpha-helix stabilizing residue;
   A6 is Gln or Glu;
   A7 is Leu or Phe;
   A8 is Met, Leu, Nle or Val;
   A10 is Gln, Glu, Asp, Ala or Val;
   A11 is Har, Arg, Ala, Ile or Lys;
   A12 is Ala, Arg or His;
   A13 is Lys, Orn, Asp, Glu, Cys, Dab or Dpr;
   A14 is Trp, Phe, Leu, Arg or His;
   A16 is Asn, Asp or absent;
   A17 is, Lys, Orn, Glu, Cys, Asp, Dab or Dpr;
R2 is OH, OR, NRH, NRR3 or NH₂, wherein each of R and R3 independently represents C1-4 alkyl (e.g. methyl); and
A13 and A17 are linked by one or more covalent bonds; and
Z1 and Z2 are independently absent, or a peptide sequence of 1-10 amino acid units selected from the group consisting of Ala, Leu, Glu, Lys, Dab, Dpr and Orn;
or a dimer, heterodimer or pharmaceutically acceptable salt or derivative thereof.

In a preferred embodiment, the present invention provides a substituted PTH(1-17) analogue peptide having the Formula III:
R1-Z1-Ac5c-Val-Aib-Glu-Ile-A6-Leu-A8-His-A10-A11-A1a-A13-A14-Leu-A16-A17-Z2-R2
   wherein
   R1 is NH₂, RHN, RR3N, wherein each of R and R3 independently represent C1-4 alkyl (e.g. methyl), acetyl, formyl, benzoyl or trifluoroacetyl; A6 is Glu or Gln;
   A8 is Met, Leu, Nle, Val;
   A10 is Gln or Glu;
   A11 is Har, Arg;
   A13 is Lys, Orn, Asp, Glu, Cys, Dab or Dpr;
   A14 is Trp, Phe,
   A16 is Asn, Asp or absent;
   A17 is Lys, Orn, Glu, Cys, Asp, Dab or Dpr;
R2 is OH, OR, NRH, NRR3 or NH₂, wherein each of R and R3 independently represents C1-4 alkyl (e.g. methyl); and
A13 and A17 are linked by one or more covalent bonds; and
Z1 and Z2 are independently absent, or a peptide sequence of 1-10 amino acid units selected from the group consisting of Ala, Leu, Glu, Lys, Dab, Dpr and Orn;
or a dimer, heterodimer or pharmaceutically acceptable salt or derivative thereof.

There are many possibilities for side-chain-to-side-chain cyclizations or bridges, including but not limited to, amides (lactams), esters (lactones), ethers, ketones or disulfides (Synthetic Peptides, A users guide. 2nd ed. 2002. Oxford University Press. Ed. Grant, G.A). Any of these possibilities may be used to covalently link the side chains of the A13 and A17 amino acid residues in the formulae defined above.

In a particularly preferred embodiment, the covalent bonding between A13 and A17 comprises a lactam bridge or a cysteine bridge.

In another embodiment of the present invention, the PTH analogue is provided in the form of a dimer. The dimer may be formed as a head-to-tail homodimer of a PTH analogue such as, but not limited to, Ac₅c-val-Aib-Glu-Ile-Gln-Leu-Met-His-Gln-Har-Ala-Lys-Trp-Leu-Asn-Asp-NH₂.

In another embodiment of the present invention, the dimer formed is a head-to-tail heterodimer of two different PTH analogues such as, but not limited to, Ac₅c-Val-Aib-Glu-Ile-Gln-Leu-Met-His-Gln-Har-Ala-Lys-Trp-Leu-Asn-Asp-NH₂ and Ac₅c-Val-Aib-Glu-Ile-Gln-Leu-Met-His-Gln-Har-Ala-Lys-Trp-Leu-Asp-NH₂.

In a further aspect, the present invention relates to a substituted PTH(1-17) peptide which comprises at least two and up to 14 substitutions relative to wild type human PTH(1-17) between residues A1 and A17 inclusive.

The peptides of Formula I, II or III preferably comprise comprises 1 or 2 substitutions at positions 1 or 3 relative to wild-type PTH, optionally in combination with 1, 2, 3, 4, 5, 6, 7, 8, 9, 10 substitutions at further positions, including 6, 7, 8, 10, 11, 12, 13, 14, 16 or 17.

Examples of combinations of residues at positions 6, 7, 8, 10, 11, 12, 13, 14, 16 or 17 which may be present in the analogues of the invention, and fall within Formulae I to II, include:

Ac₅c-Val-Aib-Glu-Ile-Gln-Leu-Met-His-Gln-Har-Ala-Lys-Trp-Leu-Asn-Asp-
NH₂
Ac₅c-Val-Aib-Glu-Ile-Glu-Leu-Met-His-Gln-Har-Ala-Lys-Trp-Leu-Asn-Asp-
NH₂
Ac₅c-Val-Aib-Glu-Ile-Gln-Leu-Leu-His-Gln-Har-Ala-Lys-Trp-Leu-Asn-Asp-
NH₂
Ac₅c-Val-Aib-Glu-Ile-Gln-Leu-Nle-His-Gln-Har-Ala-Lys-Trp-Leu-Asn-Asp-
NH₂
Ac₅c-Val-Aib-Glu-Ile-Gln-Leu-Val-His-Gln-Har-Ala-Lys-Trp-Leu-Asn-Asp-
NH₂
Ac₅c-Val-Aib-Glu-Ile-Gln-Leu-Met-His-Glu-Har-Ala-lys-Trp-Leu-Asn-Asp-
NH₂
Ac₅c-Val-Aib-Glu-Ile-Gln-Leu-Met-His-Gln-Har-Ala-Asp-Trp-Leu-Asn-Lys-
NH₂
Ac₅c-Val-Aib-Glu-Ile-Gln-Leu-Met-His-Gln-Har-Ala-lys-Phe-Leu-Asn-Asp-
NH₂

Ac5c-Val-Aib-Glu-Ile-Gln-Leu-Met-His-Gln-Har-Ala-Lys-Trp-Leu-Asp-Asp-
NH2
Ac5c-Val-Aib-Glu-Ile-Gln-Leu-Met-His-Gln-Arg-Ala-Lys-Trp-Leu-Asn-Asp-
NH2
Ac5c-Val-Aib-Glu-Ile-Gln-Leu-Met-His-Gln-Har-Ala-Cys-Trp-Leu-Asn-Cys-
NH2
Ac5c-Val-Aib-Glu-Ile-Gln-Leu-Met-His-Gln-Har-Ala-Lys-Trp-Leu-Asp-NH2

Conservative substitutions in the peptides of the invention are grouped in five Groups I to V as shown in Table 1 below where the one-letter code for natural amino acids are used:

**Table 1: Conservative substitutions of amino acids grouped by physicochemical properties. I: neutral, hydrophilic, II: acids and amides, III: basic, IV: hydrophobic, V: aromatic, bulky amino acids.**

| **I** | **II** | **III** | **IV** | **V** |
|---|---|---|---|---|
| A | N | H | M | F |
| S | D | R | L | Y |
| T | E | K | I | W |
| P | Q | | V | |
| G | | | C | |

In embodiments of the invention where Z1 is present, preferably Z1 is a straight chain oligolysine peptide, such as (Lys)₂ to (Lys)₁₀, preferably (Lys)₆.

However, in other embodiments of the invention, Z1 and/or Z2 may be absent.

In preferred peptides of the invention the terminal groups Z1 and Z2 are selected from those disclosed in WO 99/46283 as specific embodiments for "peptide sequence Z". Especially preferred is a straight oligolysine sequence, such as a hexalysine peptide sequence, (Lys)₆.

Particular peptide sequences falling within the scope of Formulae I to III are set forth in Table 3.

In a further aspect, the present invention provides an isolated nucleic acid comprising a sequence encoding a PTH analogue of the invention. The nucleic acid may be DNA or RNA, but in preferred embodiments is DNA. In a further aspect, the present invention provides an expression vector comprising the above-described nucleic acid. Typically the sequence encoding the PTH analogue is operably linked to sequences capable of driving expression of the nucleic acid, e.g. by transcription of mRNA and translation of the mRNA to protein, in a suitable host cell. In a further aspect, the present invention provides a host cell comprising nucleic acid or an expression vector as defined above.

In a further aspect, the present invention provides a method of medical treatment, comprising administering to a subject in need of treatment a PTH peptide or nucleic acid as defined herein.

In a further aspect, the present invention provides a PTH peptide or nucleic acid of the invention for use in a method of medical treatment.

In a further aspect, the present invention also provides pharmaceutical compositions comprising a PTH derivative and a pharmaceutically acceptable excipient and/or a pharmaceutically acceptable solution such as saline or a physiologically buffered solution.

In a further aspect, the present invention also provides a method for treating mammalian conditions characterized by decreases in bone mass, which method comprises administering to a subject in need thereof an effective bone mass-increasing amount of a biologically active PTH polypeptide. A preferable embodiment of the invention is drawn to conditions such as osteoporosis. The types of osteoporosis include, but are not limited to old age osteoporosis and postmenopausal osteoporosis.

In a further aspect, the present invention provides a method for determining rates of bone formation, bone resorption and/or bone modelling and remodelling comprising administering to a patient an effective amount of a labelled PTH polypeptide, for example Ac₅c-Val-Aib-Glu-Ile-Gln-Leu-Met-His-Gln-Har-Ala-Lys-Trp-Leu-Asn-Asp-NH₂ or a derivatives thereof, and determining the uptake of the peptide into the bone of the patient. The peptide is labelled with a label selected from the group consisting of: radiolabel, fluorescent label, bioluminescent label, or chemiluminescent label.

The invention is further related to a method of increasing cAMP in a mammalian cell having PTH-1 receptors, the method comprising contacting the cell with a sufficient amount of the polypeptide of the invention to increase cAMP.

### BRIEF DESCRIPTION OF THE FIGURES

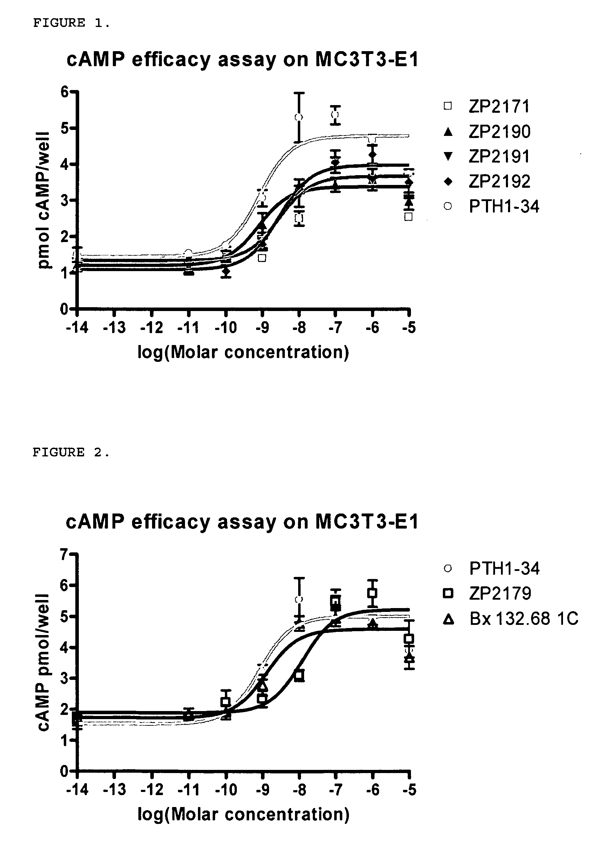

**Figure 1****:** Representative cAMP efficacy experiment on MC3T3-E1 cells with novel PTH(1-17) analogues. Cells were stimulated with varying concentrations of PTH peptides for 15 min at 37°C in the presence of a phosphodiesterase inhibitor. Error bars show standard deviation of triplicates.
**Figure 2****:** Representative cAMP efficacy experiment on MC3T3-E1 cells with novel cyclic 16-mer PTH analogues and a covalent dimer of this 16-mer. Cells were stimulated with varying concentrations of PTH peptides for 15 min at 37°C in the presence of a phosphodiesterase inhibitor. Error bars show standard deviation of triplicates.

### DETAILED DESCRIPTION

Throughout the description and claims the conventional one-letter and three-letter code for natural amino acids are used, as well as generally accepted three letter codes for other α-amino acids, such as norleucine (Nle), homoarginine (Har), 1-aminocyclopentanecarboxylic acid (Ac₅c), 2,4-diaminobytyric acid (Dab), 2,3-diaminopropionic acid (Dpr), 2,5-diaminopentanonic acid (Orn) and α-amino isobutanoic acid (Aib).

The PTH analogues of the invention contain residues which are described as being positively or negatively charged. This should be understood to mean that the side chain functionalities of the residues in question carry a whole or partial positive or negative charge at physiological pH, which is considered to be approximately 7.4

It will be understood that a single residue cannot carry a partial positive charge. This term instead refers to the average charge on the relevant residue over the whole population of peptides having the same sequence in a given system. This will be between 0 and 1 if the pK of the ionisable side chain functionality of the residue in question is within about 2 pH units of 7.4; i.e. between about 5.4 and about 9.4.

The pK of a "positively charged" residue is preferably above about 6. The pK of a "negatively charged" residue is preferably below about 8. Examples of "positively charged" residues include Lys, Arg, His, Orn, Dab and Dpr.

Examples of "negatively charged" residues include Asp and Glu.

"Neutral" residues are those which carry substantially no charge at physiological pH. These include Gln, Asn, Ala, Gly, Ser, Thr, Ile, Leu, Met, Phe, Pro, Trp, Val.

"Aromatic" residues include His, Phe, Tyr and Trp.

Conservative substitutions in the peptides of the invention are grouped in five groups I to V as shown in Table 2 below where the one-letter code for natural amino acids are used:

**Table 2: Conservative substitutions of amino acids grouped by physicochemical properties. I: neutral, hydrophilic, II: acids and amides, III: basic, IV: hydrophobic, V: aromatic, bulky amino acids.**

| **I** | **II** | **III** | **IV** | **V** |
|---|---|---|---|---|
| A | N | H | M | F |
| S | D | R | L | Y |
| T | E | K | I | W |
| P | Q | | V | |
| G | | | C | |

For reference, PTH is secreted as an 84 amino acid peptide with the following sequence H-Ser-Val-Ser-Glu-Ile-Gln-Leu-Met-His-Asn-Leu-Gly-Lys-His-Leu-Asn-Ser-Met-Glu-Arg-Val-Glu-Trp-Leu-Arg-Lys-Lys-Leu-Gln-Asp-Val-His-Asn-Phe-Val-Ala-Leu-Gly-Ala-Pro-Leu-Ala-Pro-Arg-Asp- Ala-Gly-Ser-Gln-Arg-Pro-Arg-Lys-Lys-Glu-Asp-Asn-Val-Leu-Val-Glu-Ser-His-Glu-Lys-Ser-Leu-Gly-Glu-Ala-Asp-Lys-Ala-Asp-Val-Asn-Val-Leu-Thr-Lys-Ala-Lys-Ser-Gln-OH.

The PTH analogues of the present invention have one or more amino acid substitutions, deletions, or additions compared with native PTH as defined above.

Surprisingly, a substituted PTH(1-17) molecule has been found to exhibit cAMP accumulation sustained activity towards PTH receptors, such as PTH-1 receptors.

In another aspect, the present invention provides novel peptides with both improved chemical and pharmaceutical stability against degradation compared to PTH(1-17) and [Ac₅c¹, Aib³]MPTH(1-14).

Modification at one or more of positions 6, 8, 10, 11, 13, 14, 16 or 17 by substitution with Ala, Leu, Nle, Val, Ser, Glu, Asp, Lys or Arg of [Ac₅c¹, Aib³]MPTH(1-14) increases the chemical stability of the molecule and may thus improve shelf-life and reduce degradation during formulation.

The analogue of the present invention may include chemical modification of one or more of its amino acid side chain functionalities, terminal amino group, or terminal carboxylic acid group. A chemical modification includes, but is not limited to, adding chemical moieties, creating new bonds, and removing chemical moieties. Modifications at amino acid side groups include, without limitation, acylation of lysine epsilon-amino groups, N-alkylation of arginine, histidine, or lysine, esterification of glutamic or aspartic carboxylic acid groups, and deamidation of glutamine or asparagine. Modifications of the terminal amino include, without limitation, the des-amino, N-lower alkyl, N-di-lower alkyl, and N-acyl modifications. Modifications of the terminal carboxy group include, without limitation, the amide, lower alkyl amide, dialkyl amide, and lower alkyl ester modifications. Preferably herein lower alkyl is C1-C4 alkyl. Furthermore, one or more side groups, or terminal groups, may be protected by protective groups known to the ordinarily-skilled peptide chemist.

Biological Activity of the Protein: This expression refers to the metabolic or physiologic function of the PTH analogues or derivatives thereof including similar activities or improved activities or those activities with decreased undesirable side-effects. Also included are antigenic and immunogenic activities of said compounds of said compounds or derivatives thereof.

It should be understood that the peptides of the invention might also be provided in the form of a salt or other derivative. Salts include pharmaceutically acceptable salts such as acid addition salts and basic salts. Examples of acid addition salts include hydrochloride salts, citrate salts and acetate salts. Examples of basic salts include salts where the kation is selected from alkali metals, such as sodium and potassium, alkaline earth metals, such as calcium, and ammonium ions +N (R3)3 or(R4) where R3 and R4 independently designates optionally substituted C(1-6)-alkyl, optionally substituted C(2-6)-alkenyl, optionally substituted aryl, or optionally substituted heteroaryl.

Other examples of pharmaceutically acceptable salts are described in "Remington's Pharmaceutical Sciences", Mack Publishing Company, Easton, Pennsylvania, 19th Edition, 1995, and in the Encyclopaedia of Pharmaceutical Technology.

Other derivatives of the PTH analogues of the invention include coordination complexes with metal ions such as Mn2+ and Zn2+, esters such as in vivo hydrolysable esters, free acids or bases, hydrates, prodrugs or lipids. Esters can be formed between hydroxyl or carboxylic acid groups present in the compound and an appropriate carboxylic acid or alcohol reaction partner, using techniques well known in the art. Derivatives which as prodrugs of the compounds are convertible in vivo or in vitro into one of the parent compounds. Typically, at least one of the biological activities of compound will be reduced in the prodrug form of the compound, and can be activated by conversion of the prodrug to release the compound or a metabolite of it. Examples of prodrugs include the use of protecting groups which may be removed in situ releasing active compound or serve to inhibit clearance of the drug in vivo.

In certain embodiments of the present invention, Z1 and Z2 are peptide sequence of 1-10 amino acid residues, e.g. in the range of 2-8 in particular in the range of 3-6 amino acid residues, e.g., of 2, 3, 4, 5 or 6 amino acid residues. Typically, only one of Z1 and Z2 is present, such as Z1. Each of the amino acid residues in the peptide sequence Z are independently selected from Ala, Leu, Ser, Thr, Tyr, Asn, Gln, Asp, Glu, Lys, Arg, His, Orn. Preferably, the amino acid residues are selected from Ser, Thr, Tyr, Asn, Gln, Asp, Lys, Arg, His, Orn, Dab and Dpr, especially Lys. The above-mentioned amino acids may have either D- or L-configuration, but preferably the above-mentioned amino acids have an L-configuration.

Such peptides at the N and/or C-terminus of the molecule are believed to increase solubility of the PTH analogue peptides and increase stability, e.g. against protease activity, thus leading to improved pharmacokinetic properties, such as increased half life and reduced tendency to aggregate.

Interesting peptides of the invention are shown in Table 3 below

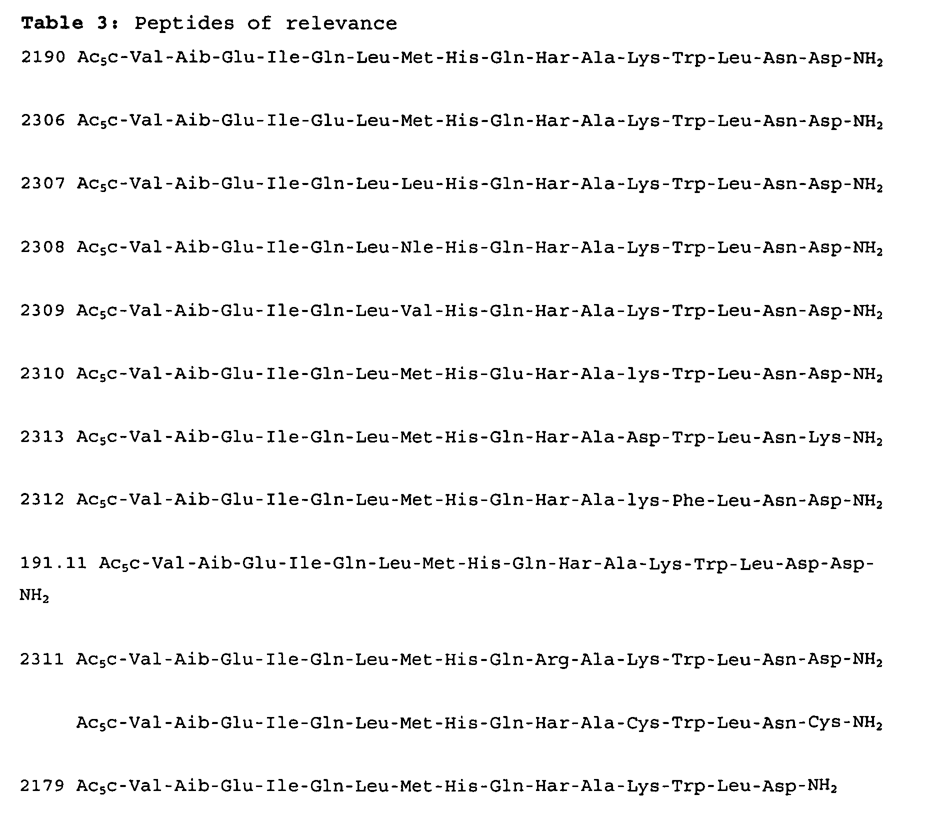

As described above, the present invention is particularly concerned with PTH analogues in which there is a covalent link between A13 and A17 of the PTH(1-17) analogues. The covalent bond has a profound effect on the potency of said peptides as compared to the similar PTH agonist with no covalent bond.

As used herein, the term covalent bond may be substituted with the terms cyclizations, links, bonds or bridges without changing the meaning of the word.

There are many possibilities for side-chain-to-side-chain cyclizations including, but not limited to, amides (lactams), esters (lactones), ethers, ketones or disulfides (Synthetic Peptides, A users guide. 2. ed. 2002. Oxford University Press. Ed. Grant, G.A).

The covalent bond between A13 and A17 comprises a lactam bridge or a cysteine bridge.

In a preferred embodiment of the present invention the lactam bond comprises

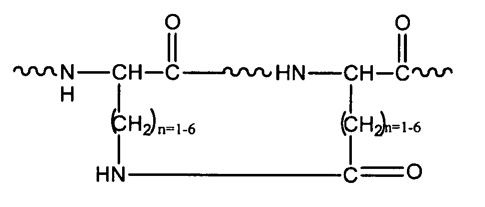

In another aspect of the invention, the lactam bond between Lys13 and Asp17 comprises a process wherein

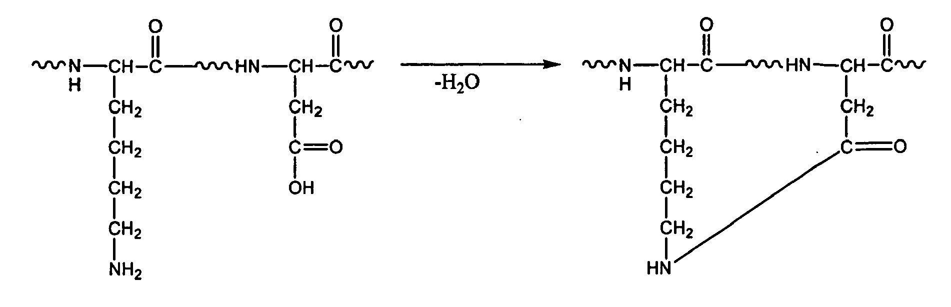

In still another aspect, the present invention relates to the formation of dimers between two PTH analogues.

In one embodiment of the present invention, the dimer formed is a head-to-tail dimer of the same PTH analogue such as Ac₅c-Val-Aib-Glu-Ile-Gln-Leu-Met-His-Gln-Har-Ala-Lys-Trp-Leu-Asn-Asp-NH₂

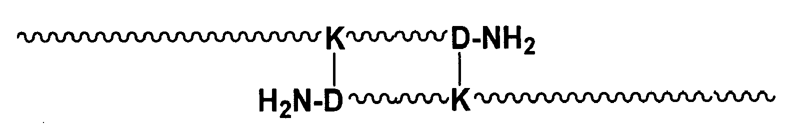

In another embodiment of the present invention, the dimer formed is a head-to-tail heterodimer of two different PTH analogues such as Ac₅c-Val-Aib-Glu-Ile-Gln-Leu-Met-His-Gln-Har-Ala-Lys-Trp-Leu-Asn-Asp-NH₂ and Ac₅c-Val-Aib-Glu-Ile-Gln-Leu-Met-His-Gln-Har-Ala-Lys-Trp-Leu-Asp-NH₂

### Indications:

The PTH analogues of the present application may be used in but not limited to the prevention or the treatment of conditions such as:
Osteoporosis, such as primary osteoporosis, endocrine osteoporosis (hyperthyroidism, hyperparathyroidism, Cushing's syndrome, and acromegaly), hereditary and congenital forms of osteoporosis (osteogenesis imperfecta, homocystinuria, Menkes' syndrome, and Riley-Day syndrome) and osteoporosis due to immobilization of extremities.
Osteomyelitis, or an infectious lesion in bone, leading to bone loss.

Hypercalcemia resulting from solid tumours (breast, lung and kidney) and hematologic malignacies (multiple myeloma, lymphoma and leukemia), idiopathic hypercalcemia, and hypercalcemia associated with hyperthyroidism and renal function disorders.

Osteopenia following surgery, induced by steroid administration, and associated with disorders of the small and large intestine and with chronic hepatic and renal diseases.

Osteonecrosis, or bone cell death, associated with traumatic injury or nontraumatic necrosis associated with Gaucher's disease, sickle cell anaemia, systemic lupus erythematosus and other conditions.

Bone loss due to rheumatoid arthritis.

Periodontal bone loss.

Osteolytic metastasis.
Bone fracture healing, and
Hyperproliferative skin disorders such as psoriasis.

The preferred indication is Osteoporosis including primary osteoporosis, endocrine osteoporosis (hyperthyroidism, hyperparathryoidism, Cushing's syndrome, and acromegaly), hereditary and congenital forms of osteoporosis (osteogenesis imperfecta, homocystinuria, Menkes' syndrome, and Riley-Day syndrome) and osteoporosis due to immobilization of extremities.

### Pharmaceutical Compositions and Administration:

The PTH analogues of the present invention, or salts or derivatives thereof, may be formulated as pharmaceutical compositions prepared for storage or administration, and which comprise a therapeutically effective amount of a PTH peptide of the present invention, or a salt or derivative thereof, in a pharmaceutically acceptable carrier.

It is within the invention to provide a pharmaceutical composition, wherein the PTH analogue, or a salt thereof is present in an amount effective to regain bone mass in a subject to whom they are administered.

As is apparent to one skilled in the medical art, a "therapeutically effective amount" of the peptides or pharmaceutical compositions of the present invention will vary depending upon the age, weight and mammalian species treated, the particular compounds employed, the particular mode of administration and the desired effects and the therapeutic indication. Because these factors and their relationship to determining this amount are well known in the medical arts, the determination of therapeutically effective dosage levels, the amount necessary to achieve the desired results described herein, will be within the ambit of the skilled person.

As used herein, "a therapeutically effective amount" is one which reduces symptoms of a given condition or pathology, and preferably which normalizes physiological responses in an individual with the condition or pathology. Reduction of symptoms or normalization of physiological responses can be determined using methods routine in the art and may vary with a given condition or pathology.

In one embodiment of the invention administration of the compounds or pharmaceutical composition of the present invention is commenced at lower dosage levels, with dosage levels being increased until the desired physiological effect is achieved. This would define a therapeutically effective amount. For the peptides of the present invention, alone or as part of a pharmaceutical composition, such doses may be between about 1 ug/kg/day to about 1000.0 ug/kg/day.

In a more preferred embodiment the effective amount of the polypeptide is about 5.0 ug/kg/day to about 500.0 ug/kg/day.

In a further preferred embodiment the effective amount of the polypeptide is about 10.0 ug/kg/day to about 400.0 ug/kg/day.

For therapeutic use, the chosen PTH analogue is formulated with a carrier that is pharmaceutically acceptable and is appropriate for delivering the peptide by the chosen route of administration. For the purpose of the present invention, the oral, rectal, nasal, or lower respiratory (pulmonal) routes are preferred. These are so-called non-parenteral routes. Certain compounds used in the present invention may also be amenable to administration by peripheral parenteral routes include intravenous, intramuscular, subcutaneous, and intra peritoneal routes of administration. The present pharmaceutical composition comprises a PTH analogue of the invention, or a salt or derivative thereof and a pharmaceutically acceptable carrier. Suitable pharmaceutically acceptable carriers are those used conventionally with peptide-based drugs, such as diluents, excipients and the like. Pharmaceutically acceptable carriers for therapeutic use are well known in the pharmaceutical art, and are described, for example, in Remington's Pharmaceutical Sciences, Mack Publishing Company, Easton, Pennsylvania, 19th Edition, 1995). pH buffering agents may be histidine, or sodium acetate. Preservatives, stabilizers, dyes and even flavouring agents may be provided in the pharmaceutical composition. For example, phenol sodium benzoate, sorbic acid and esters of p-hydroxybenzoic acid may be added as preservatives. In addition, antioxidants and suspending agents may be used, e.g. SDS, ascorbic acid, methionine, carboxy methyl cellulose, EDTA, polyethylene glycol, and Troeen.

In another embodiment, a pharmaceutically acceptable acid addition salt of the PTH peptide analogue is provided for.

The pharmaceutical compositions of the present invention may be formulated and used as tablets, capsules or elixirs for oral administration; suppositories for rectal administration; sterile solutions and suspensions for injectable administration; inhaleable formulations for nasal or pulmonal administration; and the like.

The dose and method of administration can be tailored to achieve optimal efficacy but will depend on such factors as weight, diet, concurrent medication and other factors, which those skilled in the medical arts will recognize.

When administration is non-parenteral, such as oral, rectal, nasal or pulmonary, the pharmaceutical compositions can be prepared in conventional forms. Orally administration may be in liquid formulation or as tablets or capsules. Rectal administration may be as suppositories. Nasal and pulmonary administration can be as liquid or powder.

When administration is to be parenteral, such as subcutaneous on a daily basis, injectable pharmaceutical compositions can be prepared in conventional forms, either as aqueous solutions or suspensions lyophilized, solid forms suitable for reconstitution immediately before use or suspension in liquid prior to injection, or as emulsions. Suitable excipients are, for example, water, saline, dextrose, mannitol, lactose, lecithin, albumin, sodium glutamate, and cysteine hydrochloride. In addition, if desired, the injectable pharmaceutical compositions may contain minor amounts of non-toxic auxiliary substances, such as wetting agents, or pH buffering agents. Absorption enhancing preparations (e.g., liposomes) may be utilized.

In one embodiment of the invention, the compounds are formulated for administration by infusion, e.g., when used as liquid nutritional supplements for patients on total parenteral nutrition therapy, or by injection, for example subcutaneously, intraperitoneal or intravenously, and are accordingly utilized as aqueous solutions in sterile and pyrogen-free form and optionally buffered to physiologically tolerable pH. Formulation for intramuscular administration may be based on solutions or suspensions in plant oil, e.g. canola oil, corn oil or soy bean oil. These oil based formulations may be stabilized by antioxidants e.g. BHA (butylated hydroxianisole) and BHT (butylated hydroxytoluene).

Thus, the present peptide compounds may be administered in a vehicle, such as distilled water or in saline, phosphate buffered saline, 5% dextrose solutions or oils. The solubility of the PTH analogue may be enhanced, if desired, by incorporating a solubility enhancer, such as detergents and emulsifiers.

The aqueous carrier or vehicle can be supplemented for use as injectables with an amount of gelatin that serves to depot the PTH analogue at or near the site of injection, for its slow release to the desired site of action. Alternative gelling agents, such as hyaluronic acid, may also be useful as depot agents.

The PTH analogue may be utilized in the form of a sterile-filled vial or ampoule containing a pharmacologically effective amount of the peptide, in either unit dose or multi-dose amounts. The vial or ampoule may contain the PTH analogue and the desired carrier, as an administration ready formulation. Alternatively, the vial or ampoule may contain the PTH peptide in a form, such as a lyophilized form, suitable for reconstitution in a suitable carrier, such as sterile water or phosphate-buffered saline.

The therapeutic dosing and regimen most appropriate for patient treatment will of course vary with the disease or condition to be treated, and according to the patient's weight and other parameters. Without wishing to be bound by any particular theory, it is expected that doses, in the µg/kg range, and shorter or longer duration or frequency of treatment may produce therapeutically useful results. In some instances, the therapeutic regimen may include the administration of maintenance doses appropriate for preventing tissue regression that occurs following cessation of initial treatment. The dosage sizes and dosing regimen most appropriate for human use may be guided by the results obtained by the present invention, and may be confirmed in properly designed clinical trials.

An effective dosage and treatment protocol may be determined by conventional means, starting with a low dose in laboratory animals and then increasing the dosage while monitoring the effects, and systematically varying the dosage regimen as well. Numerous factors may be taken into consideration by a clinician when determining an optimal dosage for a given subject. Such considerations are known to the skilled person.

A human dose of a PTH peptide according to the invention may in one embodiment be from about 1.0 ug/kg/day to about 1000.0 ug/kg/day, preferably from about 5.0 ug/kg/day to about 500.0 ug/kg/day, and most preferably from 10.0 ug/kg/day to about 400.0 ug/kg/day.

### Recombinant Synthesis:

The PTH peptides of the invention can also be produced as recombinant peptides by either one of the well known expression systems, such as bacteria, yeast, insect cell, mammalian cell, plant cell, or in vitro translation using standard techniques of recombinant DNA-technology (Maniatis, T., et al., In: Molecular Cloning, A Laboratory Manual, Cold Spring Harbor Press, Cold Spring Harbor, N.Y. (1982)) and peptide purification.

In one aspect, the present invention relates to an isolated nucleic acid comprising a sequence encoding a PTH analogue peptide as described above.

In one embodiment the present invention relates to an expression vector comprising the isolated nucleic acid as described above.

In another embodiment, the present invention relates to a host cell comprising a nucleic acid and expression vector as described above.

In another aspect, the present invention relates to a PTH analogue peptide or a nucleic acid as described above for use in a method of medical treatment.

In another embodiment, the present invention relates to a PTH analogue peptide or a nucleic acid as described above for the preparation of a medicament for the increase of.bone mass.

In still another embodiment, the present invention relates to a PTH analogue peptide or a nucleic acid as described above for the preparation of a medicament for the increase of bone mass in combination with a pharmaceutically acceptable carrier.

### Peptide Synthesis:

The PTH analogues may be synthesized in a number of ways including for example, a method which comprises:
(a) synthesizing the peptide by means of solid phase or liquid phase peptide synthesis and recovering the synthetic peptide thus obtained; or
(b) when the peptide is constituted by naturally occurring amino acids, expressing a nucleic acid construct that encodes the peptide in a host cell and recovering the expression product from the host cell culture; or
(c) when the peptide is constituted by naturally occurring amino acids, effecting cell-free in vitro expression of a nucleic acid construct that encodes the peptide and recovering the expression product; or
a combination of methods of (a), (b), and (c) to obtain fragments of the peptide, subsequently ligating the fragments to obtain the peptide, and recovering the peptide.

Preferred general procedures are described below. However, more detailed descriptions of solid phase peptide syntheses are found in WO 98/11125.

### Apparatus and synthetic strategy

Peptides were synthesized batch wise in a polyethylene vessel equipped with a polypropylene filter for filtration using 9-fluorenylmethyloxycarbonyl (Fmoc) as N-α-amino protecting group and suitable common protection groups for side-chain functionalities.

### Solvents

Acetonitril (HPLC-grade, Sigma-Aldrich, Germany) and NMP (N-methylpyrrolidone, Univar Europe, Denmark) was used directly without purification.

### Amino acids

Fmoc-protected amino acids were purchased from Advanced ChemTech, Kentucky, USA or Fluka, Germany, in suitable side-chain protected forms. The unnatural amino acids 1-(Fmoc-amino)-cyclopentane-1-carboxylic acid (Ac₅c), Fmoc-aminoisobutyric acid (Aib), Fmoc-Homoarg(pmc)-OH (Har) were together with Fmoc-Lys(Aloc)-OH purchased from Bachem, Germany. Fmoc-Asp(OAll)-OH was obtained from Fluka, Germany.

### Coupling reagents

Coupling reagent diisopropylcarbodiimide (DIC) was purchased from Fluka, Germany.

### Solid supports

Peptides were synthesized on TentaGel S Ram resin 0,23 mmol/g (Rapp polymere, Germany).

### Catalysts and other reagents

Diisopropylethylamine (DIEA) was purchased from Perspective Biosystem, England, piperidine and pyridine from Riedel-de Häen, Frankfurt, Germany. Ethandithiol was purchased from Aldrich/Fluka, Germany, 1-hydroxybenzotriazole (HOBt) and triisopropylsilane (TIS) from Fluka, Germany. O-(7-azabenzotriazol-1-yl)-1,1,3,3-tetramethyluronium hexafluorophosphate (HATU) was obtained from ChemPep, Miami, USA. N-methylmorpholine was purchased from Lancaster, England. Benzotriazol-1-yl-oxytripyrrolidinophosphonium hexafluorophosphate (PyBop) was obtained from Advanced ChemTech, Kentucky, USA and tetrakis(triphenylphosphine)palladium was obtained from Aldrich, Germany.

### Coupling procedures

The amino acids were coupled as *in situ* generated OBt esters made from appropriate N-α-protected amino acids and HOBt by means of DIC in NMP or as in situ generated OAt esters made from appropriate N-α-protected amino acids and HATU by means of DIEA in NMP.

### Deprotection of the N-α-amino protecting group (Fmoc).

Deprotection of the Fmoc group was performed by treatment with 20% piperidine in NMP (1x5 and 1x10 min.), followed by wash with NMP (5 x 15 ml, 5 min. each).

### Coupling of OBt-esters

3 eq. N-α-amino protected amino acid was dissolved in NMP together with 3 eq. HOBt and 3 eq DIC and then added to the resin.

### Coupling of OAt-esters

3 eq. N-α-amino protected amino acid was dissolved in NMP together with 3 eq. HATU and 3 eq DIEA and then added to the resin.

### Cleavage of peptide from resin with acid.

Peptides were cleaved from the resins by treatment with 92/1/2.5/2.5 % v/v triflouroacetic acid (TFA, Riedel-de Häen, Frankfurt, Germany)/TIS/ water/ethandithiol at r.t. for 2 h. The filtered resins were washed with 95% TFA-water and filtrates and washings evaporated under reduced pressure. The residue was precipitated with ether and freeze dried from acetic acid-water. The crude product was analyzed by high-performance liquid chromatography (HPLC) and identified by mass spectrometry (MS).

### Batchwise peptide synthesis on TentaGel resin (PEG-PS).

TentaGel S Ram resin (1g, 0.23 mmol/g) was placed in a polyethylene vessel equipped with a polypropylene filter for filtration. The resin was swelled in NMP (15ml), and treated with 20% piperidine in NMP in order to remove the initial Fmoc group on the linker TentaGel S RAM. The resin was drained and washed with NMP. The amino acids according to the sequence were coupled as preformed Fmoc-protected OBt or OAt esters (3 eq.) as described above. The couplings were continued for 2 h, unless otherwise specified. The resin was drained and washed with NMP (5 x 15 ml, 5 min each) in order to remove excess reagent. Prior to deprotection of the last Fmoc protection group, the lactam bridge was performed on the resin by first deprotection of D(OAll) and K(Aloc) and afterward cyclization as described below. After completed synthesis, cyclization and Fmoc deprotection the peptide-resin was washed with NMP (3x15 ml, 5 min each), ethanol (3x15 ml, 1 min each) and finally diethyl ether (3x15 ml, 1 min each) and dried *in vacuo.* The peptide was cleaved from the resin as described earlier and the crude peptide product was analysed and purified as described below.

### Deprotection of OAll and Aloc

5 eq. of tetrakis(triphenylphosphin)palladium was suspended in a solution comprised of 92.5/5/2.5 % v/v chloroform/acetic acid/N-methylmorpholine under a flow of N₂ for 2 min. The suspension was transferred to the peptidylresin placed in a polyethylene vessel plugged in the one end and equipped with a polypropylene filter, and the reaction was allowed to take place under a steam of N₂ 2h, at r.t. The resin was afterward drained and washed with the above mentioned solution until it turned colourless. Hereafter the resin was washed with 0.5% DIEA in NMP (3x5min.) and finally NMP (3x5min.).

### Cyclization with Lys and Asp side chains

The peptidylresin with the unprotected D and K was allowed to react with a solution of PyBop, HoBt and DIEA (3 eq. each) in NMP over night. The resin was drained and a fresh portion of the reaction mixture was added for 2 h. Finally the resin was drained and washed with NMP.

### HPLC conditions

Gradient HPLC analysis was done using a Hewlett Packard HP 1100 HPLC system consisting of a HP 1100 Quaternary Pump, a HP 1100 Autosampler a HP 1100 Column Thermostat and HP 1100 Multiple Wavelength Detector. Hewlett Packard Chemstation for LC software (rev. A.06.01) was used for instrument control and data acquisition. The following columns and HPLC buffer system was used:

| | |
|---|---|
| Column: | LiChrospher 60, 4x250mm, 10-15µm |
| Buffers: A: | 0,1% TFA in MQV; B: 0,085% TFA, 10% MQV, 90% MeCN. |
| Gradient: | 0-1,5 min. 0% B |
| | 1,5-25 min 0-50% B |
| | 25-30 min 50-100% B |
| | 30-35 min 100% B |
| | 35-40 min 100-0 % B |
| | 40-45 min 0% B |

| | |
|---|---|
| Flow 1, ml/min, oven temperature 40°C, UV detection: 1 = 215 nm. | |

### HPLC purification of the crude peptide

The crude peptide products were purified PerSeptive Biosystems VISION Workstation. VISION 3.0 software was used for instrument control and data acquisition. The following column and HPLC buffer system was used:
Column: VYDAC, C-18, 5x250mm, 10-15µm
Buffer system: Buffers: A: 0,1% TFA in MQV; B: 0,085% TFA, 10% MQV, 90% MeCN.
Gradient: 5%B-50%B over 47 min.
Flow 35 ml/min, UV detection: 1 = 215 nm and 280 nm.

### Mass spectroscopy

The peptides were dissolved in super gradient methanol (Labscan, Dublin, Ireland), milli-Q water (Millipore, Bedford, MA) and formic acid (Merck, Damstadt, Germany) (50:50:0.1 v/v/v) to give concentrations between 1 and 10 mg/ml. The peptide solutions (20 µl) were analysed in positive polarity mode by ESI-TOF-MS using a LCT mass spectrometer (Micromass, Manchester, UK) accuracy of +/- 0.1 m/z.

While the invention has been described in conjunction with the exemplary embodiments described above, many equivalent modifications and variations will be apparent to those skilled in the art when given this disclosure. Accordingly, the exemplary embodiments of the invention set forth are considered to be illustrative and not limiting. Various changes to the described embodiments may be made without departing from the spirit and scope of the invention. All documents cited herein are expressly incorporated by reference.

### EXAMPLES

### Example 1:

### Chemical Synthesis

Peptide synthesis of H-Ac₅c-Val-Aib-Glu-Ile-Gln-Leu-Met-His-Gln-Har-Ala-Lys()Trp-Leu-Asn-Asp()-NH₂ (brackets indicates sites of side chain cyclization) on TentaGel S Ram.

Dry TentaGel S Ram (0.23 mmol/g, 1.2g) was placed in a polyethylene vessel equipped with a polypropylene filter for filtration and treated as described under "Batchwise peptide synthesis on TentaGel resin" until finishing the coupling of the N-terminal Ac₅c. The three N-terminal amino acids were coupled as OAt-esters using 3 eq of HATU and DIEA (2x2h), all the other amino acids were coupled as OBt-esters as described above. After coupling the last amino acid the resin was drained, washed with NMP and Allyl and Aloc were deprotected as described under "Deprotection of OAll and Aloc" and the peptide was cyclized as described under "Cyclization with Lys and Asp side chains". After cyclization the last N-terminal Fmoc group was deprotected and the resin was washed with NMP, EtOH and ether and dried in vacuo. The peptide was cleaved from the resin as described above. The crude product was analyzed by HPLC and MS and the purity was found to be 24% and the identity of the peptide was confirmed by MS (found MH⁺ 2071.13, calculated MH⁺ 2071.11). Yield of crude product 259 mg. The peptide was purified to 96% as described above.

### Synthesis of dimers

Example of synthesizing the head-to-tail dimer of peptide 2190 Ac₅c-Val-Aib-Glu-Ile-Gln-Leu-Met-His-Gln-Har-Ala-Lys-Trp-Leu-Asn-Asp-NH₂: The dimer is synthesized on a TentaGel S Ram resin, and the amino acids are coupled in the following Order: D(OAll)-Asn-Leu-Trp-Lys-(Dde-Lys(Fmoc))-(Fmoc-Asp-NH₂)-Asn-Leu-Trp-(Dde-Lys(Fmoc))-Ala-Har-Gln-His-Met-Leu-Gln-Ile-Glu-Aib-Val-Ac₅c. After coupling of the second Dde-Lys(Fmoc) the cyclization between Asp and Lys are performed according to the description for the monomer peptide synthesis. Afterward the Dde protection groups on Lys are removed by agitating the peptidylresin in 2% hydrazine in NMP (3x5 min.). The following amino acids are then coupled to the free amino group on K in 6 equivalents. Cleavage of the peptide from the resin and the further processing of the dimer are performed as described for the monomer peptide synthesis.

### Example 2

### In vitro testing of PTH analogues in MC3T3-E1 cAMP efficacy assay

### Materials and methods

MC3T3-E1 subclone 4 (mineralizing) (MC3T3-E1) cells (ATCC CRL-2593) were grown in α-MEM (Invitrogen #32571)/10% fetal calf serum + penicillin/streptomycin in a humid atmosphere of 95% air/5% CO₂ at 37°C.

Peptides were dissolved in phosphate buffered saline and further diluted in Tyrode's buffer (TB, Sigma, T2145) containing 0.1% alkali-treated casein (ATC) (Livesey and Donald, Clin. Chim. Acta 1982, 123: 193-8.) and 100µM isobutyl-methyl-xanthine, IBMX, Sigma I5879).

[¹²⁵I]cAMP FlashPlate® assays (cat. no. # SMP100 1A, Perkin Elmer Life Sciences) were used to determine cAMP concentrations.

MC3T3-E1 cells were seeded at 10,000 cells/well in 96-microtiter plates and grown overnight before the efficacy assays. On the day of analysis the growth medium was carefully removed by suction. Cells were washed once with 200 µl TB/0.1% ATC. The buffer was replaced with 100µl reaction mixture (± test peptide, +100 µM IBMX) and incubated at 37° C for 13 min. The reaction was stopped by addition of 25 µl of ice-cold 0.5 M HCl. Cells were in the following incubated on ice for 60 min. 75 µl acetate-buffer were added to each well in a 96-well cAMP FlashPlate. 25 µl of the acid cell extract and 100 µl [¹²⁵I] cAMP solution were added onto the same FlashPlate. FlashPlates were incubated overnight at 4°C, emptied by suction and counted in a TopCounter.

### Study design

Determinations were performed in triplicates at all doses. Standards were included as single determinations, duplicates or triplicates in each experiment, preferentially on each plate.
Concentration levels and groups
Efficacy was evaluated in the concentration range from 10 pM to 10 µM.

### Data analysis and statistics

Concentration-dependent cAMP responses were imported into GraphPad Prism vers. 4, transformed and plotted. Curves were fitted with a function for sigmoidal dose response curve non-linear fit Y=Bottom + (Top-Bottom)/(1+10^((LogEC50-X))) where X is the logarithm of concentration and Y is the response. Y starts at Bottom and goes to Top with a sigmoid shape. pEC50 and the maximally inducible concentration of cAMP were evaluated.

Overall statistical differences were analyzed using two-way ANOVA. Post-hoc comparisons were made by the use of Fisher's Least Significance Test. Results were considered significant, when p was lower than 0.05.

### RESULTS

MC3T3-E1 cells were analysed by PCR and shown to express mRNA for the PTH receptor 1 (data not shown).The positive reference compound PTH(1-34) induced a robust cAMP response in this cell line (data not shown).

Several peptides were compared in the cAMP efficacy assay t (Table 4).

**Table 4: Peptides tested in the MC3T3-E1 assay including the dimmer 2179**

| **ZP ID** | **Sequence** |
|---|---|
| **94T= PTH(1-34) Positive control reference compound** | |
| **2171T** | H-Ac5c-Val-Aib-Glu-Ile-Gln-Leu-Met-His-Gln-Har-Ala-Lys-Trp-NH2 |
| **2179T** | Cyclic H-Ac5c-Val-Aib-Glu-Ile-Gln-Leu-Met-His-Gln-Har-Ala-Lys-Trp-Leu- Asp-NH2 |
| **2179 dimerT** | Dimer H-Ac5c-Val-Aib-Glu-Ile-Gln-Leu-Met-His-Gln-Har-Ala-Lys-Trp-Leu- Asp-NH2 |
| **2190T** | Cyclic H-Ac5c-Val-Aib-Glu-Ile-Gln-Leu-Met-His-Gln-Har-Ala-Lys-Trp-Leu- Asn-Asp-NH2 |
| **2191T** | H-Ac5c-Val-Aib-Glu-Ile-Gln-Leu-Met-His-Gln-Har-Ala-Lys-Trp-Leu-Asn-Asp- NH2 |
| **2192T** | H-Ac5c-Val-Aib-Glu-Ile-Gln-Leu-Met-His-Gln-Har-Ala-Lys-Trp-Leu-Asn-Asn- NH2 |

The four short and potent PTH receptor were found to induce full agonism responses on cAMP accumulation with EC50 values similar to the reference compound PTH(1-34), (Table 5).

**Table 5: pEC50 and maximum efficacy values ± SEM of novel PTH analogues in the MC3T3-E1 cAMP efficacy assay. Values were compared to PTH(1-34). *: p<0.05 (Fisher LSD-test); n= number of determinations.**

| compound | pEC50 ± SEM | Emax ± SEM [pmol/well] | n ₅ |
|---|---|---|---|
| ZP2190 | 9.0 ± 0.2 | 4.9 + 0.8 | 4 |
| ZP2191 | 8.2 ± 0.2 * | 5.7 ± 1.0 | 4 |
| ZP2192 | 8.6 ± 0.1 * | 5.5 ± 0.9 | 4¹⁰ |
| PTH(1-34) | 9.1 ± 0.1 | 4.4 ± 0.4 | 4 |
| ZP2171 | 8.4 ± 0.1 * | 4.5 ± 1.0 | 3₁₅ |
| ZP2179 | 8.0 ± 0.1 * | 5.0 ± 0.2 | 4 |
| ZP2179 dimer | 8.9 ± 0.1 | 5.0 ± 0.3 | 4 |

All tested peptides were full agonists on PTH receptor cAMP signaling. Their dose response curves could be fitted by a simple sigmoid curve (Hill coefficient close to 1). All data were included in the analysis, i.e. no outliers were removed.

PTH(1-34) induced cAMP with an EC50 value around 1 nM, consistent with published reports on similar assay systems (Rhee et. al., Yonsei Med. J. 2006, 47: 214-22.;Murrills et. al., Bone 2004, 35: 1263-72.). ZP2171 was a three- to fourfold weaker agonist than PTH(1-34).

ZP2190, the cyclic PTH(1-17) analogue containing a covalent bond between K13 and D17, was the most potent molecule among the new, short PTH analogues. ZP2190 had an EC50 comparable to PTH(1-34) and it was significantly more potent than ZP2171, confirming the importance of amino acids 15 to 17 for agonist activity.

ZP2191, a linear PTH(1-17) variant of ZP2190 containing the same K13/D17 combination of amino acids able to form a salt-bridge in the C-terminus, was a 6-fold weaker agonist than ZP2190.
The linear analogue ZP2192, a ZP2191 variant without salt-bridge forming capabilities, was 2-3 fold weaker than ZP2190.

The cyclic PTH-16mer ZP2179 was a tenfold weaker agonist than PTH(1-34) in MC3T3-E1 cells. However, a covalent dimer of ZP2179 turned into a stronger agonist, with indistinguishable potency to PTH(1-34) and ZP2190.

### Conclusion

We have identified four novel, short peptide PTH receptor 1 mimetics that exert full agonist responses in an osteoblast-like cell line. Among these the 17mer cyclized molecules ZP2190 and dimeric ZP2179were the most potent compounds with an EC50 around 1 nM which is similar to PTH(1-34). K13-D17 cyclization was associated with increased potency as illustrated by 2-6 fold lower potency of the linear analogues ZP2191 and ZP2192.

### Example 3

### In vivo testing in OVX rat model:

The ovariectomiced(OVX) rat may be used to test the effect of the PTH analogues on osteopenia/osteoporosis in vivo. The OVX rat develop osteopenia due to ovarian hormone deficiency. Osteopenia can be detected as early as 14 days post OVX, increase for the next 100 days and then stabilized (Wronski TJ, Cintron M, Dann LM. Temporal relationship between bone loss and increased bone turnover in ovariectomized rats. Calcif Tissue Int 1988; 43(3):179-183).
The OVX rat model is considered the "golden standard" by both authorities and industry as a model for osteoporosis (Peter C, Rodan GA. Preclinical safety profile of alendronate. Int J Clin Pract Suppl 1999; 101:3-8, and Stewart AF, Cain RL, Burr DB, Jacob D, Turner CH, Hock JM. Six-month daily administration of parathyroid hormone and parathyroid hormone-related protein peptides to adult ovariectomized rats markedly enhances bone mass and biomechanical properties (A comparison of human parathyroid hormone 1-34, parathyroid hormone-related protein 1-36, and SDZ-parathyroid hormone 893. J Bone Miner Res 2000; 15(8):1517-1525).

The study is built on a placebo controlled, active controlled comparative design using nullipara, ovariectomiced rats. Treatment with test compound is initiated 4-8 weeks after OVX surgery where the progressive osteopenia/osteoporosis is shown to be severe. After 4-8 weeks of treatment the animals are sacrificed. Spine, tibia and femur are collected for analysis of bone mass density (BMD) by DEXA-scan and bone strength measurements. Bone strength measurement is performed for instance as three point bending of femur, and/or as compression of the femoral neck and vertebrae, to determine the strength in both predominantly cortical and predominantly trabecular bone.

## Claims

1. A biologically active PTH(1-17)analogue peptide represented by Formula I which consists of:
R1-Z1-A1-A2-A3-A4-A5-A6-A7-A8-A9-A1a-A11-A12-A13-A14-Leu-A16-A17-Z2-R2
wherein
R1 is NH₂, RHN, RR3N, wherein each of R and R3 independently represent C1-4 alkyl (e.g. methyl), acetyl, formyl, benzoyl or trifluoroacetyl;
A1 is Ac5c, Gly, Ser, Ala or any alpha-helix stabilizing residue;
A2 is Val or a conservative substitution;
A3 is Aib, Ala, Ser or any alpha-helix stabilizing residue;
A4 is Glu or a conservative substitution;
A5 is Ile or a conservative substitution;
A6 is Gln, Glu or a conservative substitution;
A7 is Leu or Phe or a conservative substitution;
A8 is Met, Leu, Nle, Val or a conservative substitution;
A9 is His or a conservative substitution;
A10 is Gln, Glu, Asp, Ala, Val or a conservative substitution;
A11 is Har, Arg, Ala, Ile, Lys or a conservative substitution;
A12 is Ala, Arg, His or a conservative substitution;
A13 is Lys, Orn, Asp, Glu, Cys, Dab or Dpr;
A14 is Trp, Phe, Leu, Arg, His or a conservative substitution;
A16 is Asn, Asp, a conservative substitution or absent;
A17 is, Lys, Orn, Glu, Cys, Asp, Dab or Dpr; and
R2 is OH, OR, NRH, NRR3 or NH₂, wherein each of R and R3 independently represents C1-4 alkyl (e.g. methyl); and
A13 and A17 are linked by one or more covalent bonds; and
Z1 and Z2 are independently absent, or a peptide sequence of 1-10 amino acid units selected from the group consisting of Ala, Leu, Met, Gln, Glu, Lys, Dab, Dpr and Orn;
or a dimer, heterodimer, or pharmaceutically acceptable salt or derivative thereof.

2. A biologically active PTH(1-17) analogue peptide represented by Formula II which consists of:
R1-Z1-A1-Val-A3-Glu-Ile-A6-A7-A8-His-A10-A11-A12-A13-A14-Leu-A16-A17-Z2-R2
wherein
R1 is NH₂, RHN, RR3N, wherein each of R and R3 independently represent C1-4 alkyl (e.g. methyl), acetyl, formyl, benzoyl or trifluoroacetyl;
A1 is Ac5c, Gly, Ser, Ala or any alpha-helix stabilizing residue;
A3 is Aib, Ala, Ser or any alpha-helix stabilizing residue;
A6 is Gln or Glu;
A7 is Leu or Phe;
A8 is Met, Leu, Nle or Val;
A10 is Gln, Glu, Asp, Ala or Val;
A11 is Har, Arg, Ala, Ile or Lys;
A12 is Ala, Arg or His;
A13 is Lys, Orn, Asp, Glu, Cys, Dab or Dpr;
A14 is Trp, Phe, Leu, Arg or His;
A16 is Asn, Asp or absent;
A17 is, Lys, Orn, Glu, Cys, Asp, Dab or Dpr;
R2 is OH, OR, NRH, NRR3 or NH₂, wherein each of R and R3 independently represents C1-4 alkyl (e.g. methyl); and
A13 and A17 are linked by one or more covalent bonds; and
Z1 and Z2 are independently absent, or a peptide sequence of 1-10 amino acid units selected from the group consisting of Ala, Leu, Lys, Dab, Dpr and Orn;
or a dimer, heterodimer, or pharmaceutically acceptable salt or derivative thereof.

3. A biologically active PTH(1-17) analogue peptide represented by Formula III which consists of:
R1-Z1-Ac5c-Val-Aib-Glu-Ile-A6-Leu-A8-His-A10-A11-Ala-A13-A14-Leu-A16-A17-Z2-R2
wherein
R1 is NH₂, RHN, RR3N, wherein each of R and R3 independently represent C1-4 alkyl (e.g. methyl), acetyl, formyl, benzoyl or trifluoroacetyl;
A6 is Glu or Gln;
A8 is Met, Leu, Nle, Val;
A10 is Glnor Glu;
A11 is Har, Arg;
A13 is Lys, Orn, Asp, Glu, Cys, Dab or Dpr;
A14 is Trp, Phe,
A16 is Asn, Asp;
A17 is Lys, Orn, Glu, Cys, Asp, Dab or Dpr;
R2 is OH, OR, NRH, NRR3 or NH₂, wherein each of R and R3 independently represents C1-4 alkyl (e.g. methyl); and
A13 and A17 are linked by a covalent bond; and
Z1 and Z2 are independently absent, or a peptide sequence of 1-10 amino acid units selected from the group consisting of Ala, Leu, Lys, Dab, Dpr and Orn;
or a dimer, heterodimer or pharmaceutically acceptable salt or derivative thereof.

4. The PTH(1-17) analogue peptide according to any one of claims 1 to 3, wherein the one or more covalent bonds between the amino acid residues at position 13 and position 17 comprises a lactam bridge or a cysteine bridge.

5. The PTH(1-17) analogue peptide according to claim 4, wherein the covalent bond between position 13 and position 17 is a lactam bridge.

6. The PTH(1-17) analogue peptide according to claim 4, wherein the one or more covalent bonds are formed between two PTH analogues.

7. The PTH(1-17) analogue peptide according to any one of the preceding claims which comprises between two and 14 substitutions relative to wild type human PTH(1-17) between residues A1 and A17 inclusive.

8. The PTH(1-17) analogue peptide according to any one of the preceding claims which comprises 1 or 2 substitutions at positions 1 or 3 relative to wild-type PTH, optionally in combination with 1, 2, 3, 4, 5, 6, 7, 8, 9, 10 substitutions at further positions, including 6, 7, 8, 10, 11, 12, 13, 14, 16 or 17.

9. The PTH(1-17) analogue peptide according to any one of the preceding claims which comprises substitutions at positions 1 to 10 relative to wild type PTH, optionally in combination with substitutions at one or more further positions, including 11, 12, 13, 14, 16 or 17.

10. The PTH(1-17) analogue peptide according to any one of the preceding claims which comprises substitutions at position 13 with Asp, Lys, Orn, Glu, or Cys and/or substitution of position 17 with Lys, Asp, Orn, Glu or Cys.

11. The PTH(1-17) analogue peptide according to any one of claim 1 to 10 wherein Z1 is an oligolysine selected from (Lys)₂ to (Lys)₁₀, preferably (Lys)₆.

12. The PTH(1-17) analogue peptide according to any one of claims 1 to 11 comprising one of the following combinations of residues: 
Ac₅c-Val-Aib-Glu-Ile-Gln-Leu-Met-His-Gln-Har-Ala-Lys-Trp-Leu-Asn-Asp-
NH₂;
Ac₅c-Val-Aib-Glu-Ile-Glu-Leu-Met-His-Gln-Har-Ala-Lys-Trp-Leu-Asn-Asp-
NH₂;
Ac₅c-Val-Aib-Glu-Ile-Gln-Leu-Leu-His-Gln-Har-Ala-Lys-Trp-Leu-Asn-Asp-
NH₂;
Ac₅c-Val-Aib-Glu-Ile-Gln-Leu-Nle-His-Gln-Har-Ala-Lys-Trp-Leu-Asn-Asp-
NH₂;
Ac₅c-Val-Aib-Glu-Ile-Gln-Leu-Val-His-Gln-Har-Ala-Lys-Trp-Leu-Asn-Asp-
NH₂;
Ac₅c-Val-Aib-Glu-Ile-Gln-Leu-Met-His-Glu-Har-Ala-lys-Trp-Leu-Asn-Asp-
NH₂;
Ac₅c-Val-Aib-Glu-Ile-Gln-Leu-Met-His-Gln-Har-Ala-Asp-Trp-Leu-Asn-Lys-
NH₂;
Ac₅c-Val-Aib-Glu-Ile-Gln-Leu-Met-His-Gln-Har-Ala-lys-Phe-Leu-Asn-Asp-
NH₂;
Ac₅c-Val-Aib-Glu-Ile-Gln-Leu-Met-His-Gln-Har-Ala-Lys-Trp-Leu-Asp-Asp-
NH₂;
Ac₅c-Val-Aib-Glu-Ile-Gln-Leu-Met-His-Gln-Arg-Ala-Lys-Trp-Leu-Asn-Asp-
NH₂;
Ac₅c-Val-Aib-Glu-Ile-Gln-Leu-Met-His-Gln-Har-Ala-Cys-Trp-Leu-Asn-Cys-
NH₂;
Ac₅c-Val-Aib-Glu-Ile-Gln-Leu-Met-His-Gln-Har-Ala-Lys-Trp-Leu-Asp-NH₂;
 and N-terminally acetylated form thereof, a dimmer or heterodimer or a pharmaceutically acceptable salt and derivative thereof.

13. An isolated nucleic acid comprising a sequence encoding a PTH analogue peptide according to any one of the preceding claims.

14. An expression vector comprising the isolated nucleic acid of claim 13 operably linked to sequences to control its expression.

15. A host cell comprising nucleic acid according to claim 12 or an expression vector according to claim 14.

16. A PTH analogue peptide according to any one of claims 1 to 12 or a nucleic acid according to claim 13 for use in a method of medical treatment.

17. Use of a PTH analogue peptide according to any one of claims 1 to 12 or nucleic acid according to claim 13 in the preparation of a medicament for the increase of bone mass.

18. A pharmaceutical composition comprising a PTH analogue peptide according to any one of claims 1 to 12 or nucleic acid according to claim 13, in combination with a pharmaceutically acceptable carrier.
